# FASCICULE DE BREVET EUROPEEN

(11) **EP 1 848 374 B1**
(45) Date de publication et mention de la délivrance du brevet: **07.03.2012**
(21) Numéro de dépôt: 06709415.1
(22) Date de dépôt: 20.01.2006
(51) Int. Cl.: A61F 2/16

(54) **INJECTEUR D'IMPLANT INTRAOCULAIRE SOUPLE**
FLEXIBLER INTRAOKULARIMPLANTAT-INJEKTOR
FLEXIBLE INTRAOCULAR IMPLANT INJECTOR

(30) Priorité: 21.01.2005 FR 0500633
(43) Date de publication de la demande: 31.10.2007
(73) Titulaire: Corneal Innovation, 75002 Paris (FR)
(72) Inventeur: QUINTIN, Nicolas, F-74370 Villaz (FR); VINCHON, Cyrille, F-74270 Chilly (FR); ANDRE, Jean-Marc, F-73290 La Motte Servolex (FR)
(74) Mandataire: Schwarz & Partner
(86) Numéro de dépôt international: PCT/FR2006/050030
(87) Numéro de publication internationale: WO 2006/077349

(56) Documents cités:
- EP-A- 0 270 257
- EP-A- 0 363 213
- EP-A- 1 360 946
- WO-A-97/15253
- WO-A-2004/026106
- FR-A- 2 814 360

## Description

La présente invention a pour objet un injecteur d'implant intraoculaire souple.

De tels injecteurs d'implant souple sont décrits notamment dans les demandes de brevet WO 01/87186 et WO 02/26167 au nom de la société CORNEAL INDUSTRIE.

Les implants intraoculaires souples sont des implants dans lesquels au moins la partie optique est réalisée à partir d'un matériau deformable tel que des acryliques hydrophiles ou hydrophobes. La partie optique et également la partie haptique de ces implants peuvent être pliées ou enroulées de telle manière que dans cet état, l'implant puisse être introduit dans l'oeil à travers une incision de petite dimension qui était typiquement de l'ordre de 3 mm.

Pour faciliter le travail du chirurgien, on a mis au point des dispositifs appelés injecteurs d'implant souple qui permettent d'une part, le pliage de l'implant, et d'autre part, la mise en place à l'intérieur de l'oeil de l'implant plié à l'aide d'une canule introduite dans l'incision réalisée dans la paroi de vieil.

L'injecteur se compose essentiellement d'une chambre de pliage dans laquelle l'implant non plié peut être mis en place et dans laquelle, par manoeuvre d'un organe mécanique, on obtient un enroulement correct ou un pliage correct de l'implant. Cet injecteur comporte également un canal interne formant canule dans lequel l'implant plié peut être déplacé à l'aide d'un piston jusqu'à l'extrémité ouverte de la canule qui est disposée à l'intérieur de l'oeil. Lorsque le piston arrive en bout de course, l'implant est éjecté à l'intérieur de l'oeil à l'endroit convenable et spontanément il reprend sa forme initiale.

Pour diminuer encore la dimension de l'incision pratiquée dans la paroi de l'oeil, on a développé des injecteurs d'implants dans lesquels l'extrémité de la canule disposée à l'intérieur de l'oeil a un diamètre encore réduit par rapport à celui de la chambre de pliage. Ainsi, en poussant l'implant déjà plié dans le canal de la canule, on réduit encore le diamètre externe de l'implant plié Ce type d'injecteur soulève un certain nombre de problèmes en ce qui concerne l'action du piston sur l'implant plié en bout de course.

Au moins l'extrémité de l'injecteur introduit dans l'oeil du patient doit être stérilisée après usage. Pour éviter les problèmes et risques liés à la stérilisation plus ou moins bien effectuée, on a mis sur le marché des injecteurs réalisés à partir de matériaux plastiques qui sont non réutilisables puisque non stérilisables efficacement.

L'invention selon WO 97/15253 concerne un appareil d'injection de cristallin artificiel déformable présentant un piston mobile (112) avec une extrémité déformable (118), de préférence une extrémité de piston déformable élastiquement. Dans un mode préféré de réalisation, l'extrémité de piston est réalisée dans un matériau souple déformable élastiquement pour ne pas endommager la surface ou l'intérieur du cristallin artificiel déformable, poussé à travers un passage de distribution de l'appareil d'injection susmentionné.

Dans certaines circonstances d'utilisation d'un tel injecteur, il est important de rendre celui-ci effectivement et définitivement non réutilisable après une première utilisation de celui-ci.

Un objet de la présente invention est de fournir un injecteur d'implant souple qui permet, d'une part, un pliage de l'implant pour conférer à celui-ci des dimensions encore réduites et qui, d'autre part, soit effectivement non réutilisable après une première utilisation.

Pour atteindre ce but selon l'invention, l'injecteur d'implant souple comprend un canal comportant une partie courante de diamètre D1, une partie d'extrémité ouverte de diamètre D2 (avec D2 < D1) et une partie de raccordement de forme sensiblement tronconique pour raccorder ladite partie courante à ladite partie d'extrémité, un piston rigide déplaçable dans le canal pour pousser l'implant plié dans le canal, une pièce de poussée sensiblement de révolution, ladite pièce de poussée ayant, en l'absence de contraintes, un diamètre externe D3 au moins égal à, D1, ledit injecteur est caractérisé en ce que le pièce de poussée sensiblement de révolution réalisée en un matériau hydrophobe et élastiquement déformable, ladite pièce étant disposée dans ledit canal entre l'implant plié et l'extrémité dudit piston sans liaison mécanique avec ledit piston, par quoi, lorsque l'implant est poussé dans le canal à l'aide du piston, ladite pièce de poussée peut, par déformation élastique, pénétrer en partie dans la partie d'extrémité du canal en expulsant l'implant hors du canal, ladite pièce de poussée restant coincée dans ladite partie d'extrémité du canal lors du retrait du piston, rendant l'injecteur non réutilisable.

Par "sans liaison mécanique avec le piston" il faut comprendre qu'il n'existe aucun moyen de solidarisation mécanique entre extrémité du piston et la pièce de poussée qu'il s'agisse d'un collage d'un emboîtement, d'un surmoulage, etc.. La pièce de poussée est totalement libre par rapport au piston et elle est simplement poussée par le piston par simple contact entre l'extrémité du piston et la face postérieure de la pièce de poussée.

On comprend que grâce au fait que l'extrémité du canal de l'injecteur présente un diamètre réduit par rapport à celui de la partie courante qui correspond au diamètre de la chambre d'enroulement ou de pliage, on obtient un pliage de l'implant plus poussé et donc de dimension plus réduite de l'implant. En outre, du fait de la présence de la pièce de poussée élastiquement déformable, l'implant peut être poussé effectivement y compris dans la partie terminale du canal de l'injecteur de diamètre réduit, ce qui assure l'éjection correcte de l'implant à l'intérieur de l'oeil du patient. En outre, on comprend que du fait que la pièce de poussée n'est en aucun cas liée mécaniquement au piston lorsqu'à la fin de la course du piston la pièce de poussée déformable a été introduite dans l'extrémité de diamètre réduit du canal de l'injecteur, cette pièce y reste coincée lorsque le chirurgien procède au retrait du piston. Le coincement de la pièce de poussée dans l'extrémité du canal de l'injecteur rend celui-ci totalement non réutilisable.

Enfin, du fait que la pièce de poussée n'est pas mécaniquement liée au piston, la déformation radiale de la pièce de poussée est homogène et régulière.

Selon un mode préféré de mise en oeuvre, l'extrémité de la pièce de poussée destinée à coopérer avec l'extrémité du piston a un diamètre D4 > D1 et l'autre extrémité de la pièce de poussée destinée à coopérer avec l'implant plié a un diamètre D5 sensiblement égal à D1, par quoi la déformation élastique radiale de la première extrémité de la pièce de poussée crée entre elle-même et la paroi interne du canal un coefficient de frottement.

On comprend que grâce au fait que l'extrémité postérieure, c'est-à-dire tournée vers le piston de la pièce de poussée, a un diamètre externe sensiblement supérieur à celui de la paroi interne du canal, cette extrémité inférieure est légèrement comprimée. Cela assure ainsi un raclage de la paroi interne du canal même si celle-ci comporte certaines aspérités. Ce raclage permet d'éviter le coincement des anses haptiques entre la pièce de poussée et la paroi interne du canal. En outre, cette compression de la partie postérieure de la pièce de poussée assure un coefficient de frottement constant entre les pièces de poussée et la paroi interne du canal, ce qui facilite le travail du chirurgien pour obtenir un déplacement régulier de l'implant dans le canal notamment dans la phase finale de la poussée permettant l'éjection de l'implant hors de l'extrémité de l'injecteur.

D'autres caractéristiques et avantages de l'invention apparaîtront mieux à la lecture de la description qui suit d'un mode préféré de réalisation de l'invention donné à titre d'exemple non limitatif.
- La figure 1 est une vue d'ensemble en perspective d'un injecteur d'implant souple ;
- les figures 2A à 2D sont des vues en coupe longitudinale de la partie antérieure de l'injecteur, qui illustrent l'utilisation de l'injecteur ; et
- la figure 3 est une vue de côté de la pièce de poussée.

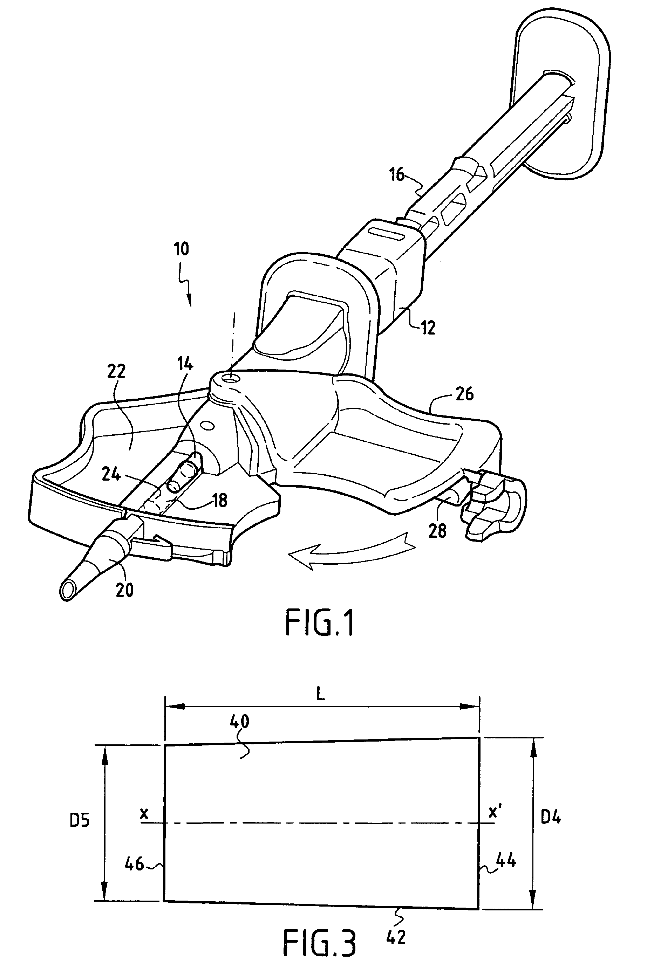

En se référant à la figure 1, on va décrire l'ensemble d'un injecteur pour implant souple du type considéré. L'injecteur 10 comprend un corps 12 définissant un canal 14. Le canal 14 comprend une partie postérieure 15 de guidage d'un piston 16, une partie intermédiaire 18 constituant une chambre de pliage de l'implant souple et une partie antérieure 20 constituant une canule. La chambre de pliage 18 comporte une partie fixe 22 définissant une première moitié de mâchoire 24 et une partie mobile 26 définissant une deuxième partie de mâchoire de pliage 28.

Lorsque l'on a mis en place un implant non plié dans la chambre de pliage 18 et que l'on rapproche la partie mobile 26 de la partie fixe 22, on obtient un pliage progressif de l'implant souple qui, à la fin de cette opération, se trouve plié dans la chambre de pliage 18.

Une fois que l'on a réalisé le pliage de l'implant souple dans la chambre 18, à l'aide du piston 16, on pousse l'implant plié dans la canule 20 qui a été introduite dans l'incision pratiquée dans l'oeil du patient de telle manière que l'implant soit introduit dans l'oeil de celui-ci.

La description précédente correspond à l'organisation générale d'un injecteur d'implant souple connu.

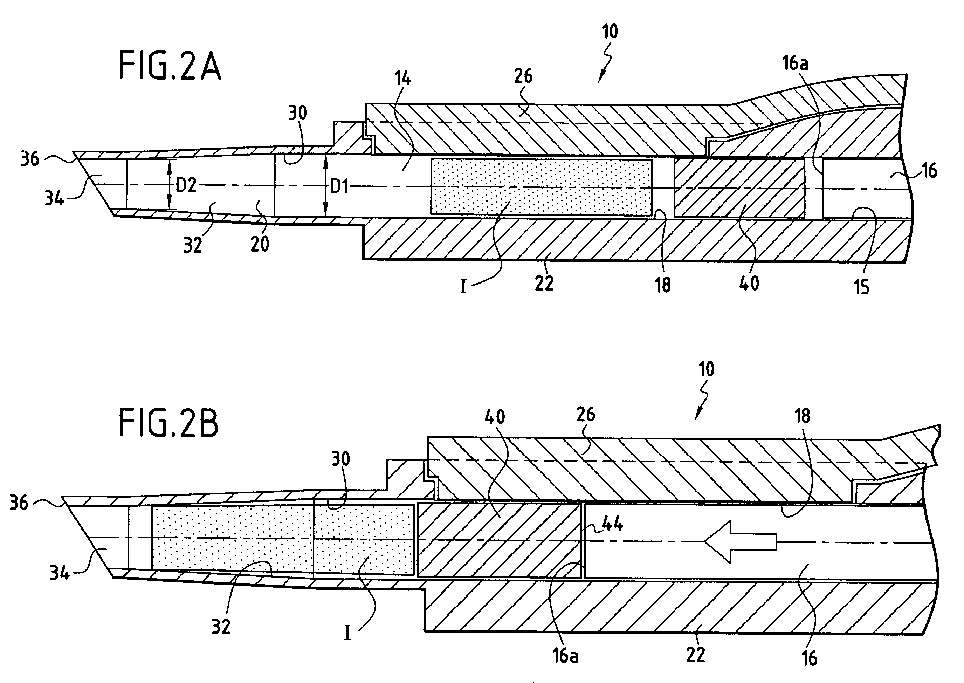

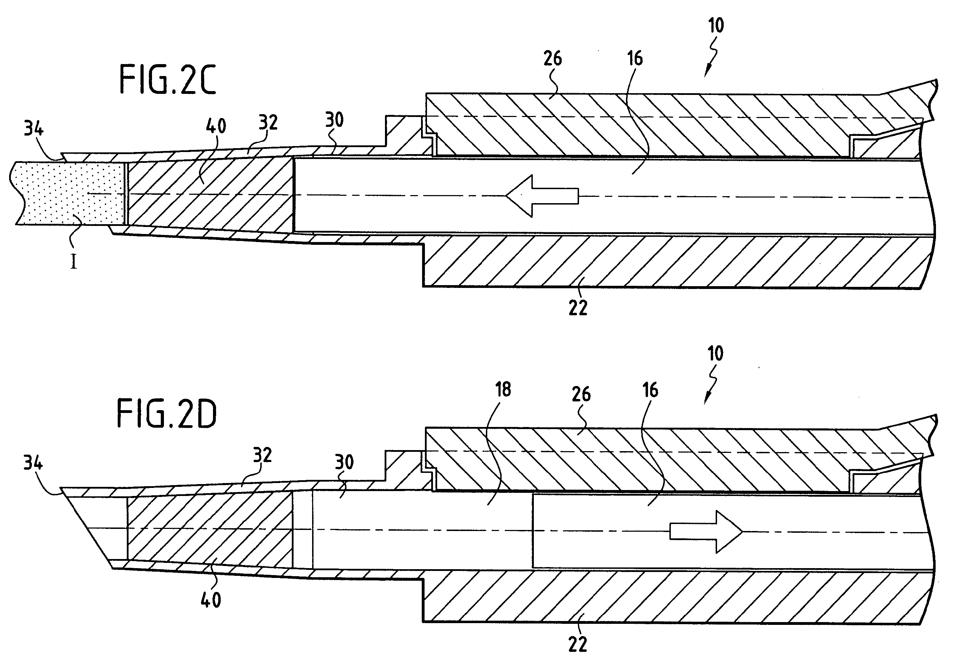

En se référant maintenant aux figures 2A à 2D et à la figure 3, on va décrire le type particulier d'injecteur d'implants, objet de la présente invention.

Sur la figure 2A, on a représenté la partie antérieure de l'injecteur d'implants 10. On retrouve la chambre de pliage 18 définie par la mâchoire fixe 22 et la mâchoire mobile 26. On retrouve également le canal 14 qui est constitué par une partie postérieure 15 de guidage du piston 16, par la chambre de pliage 18 et par une partie antérieure formant canule 20.

Comme le montre la figure 2A, la partie antérieure 20 du canal comporte une première portion 30 raccordée à la sortie de la chambre de pliage et présentant un diamètre D1 qui est le diamètre courant du canal 14, une portion intermédiaire de raccordement 32 dont le diamètre interne varie de D1 à D2, bien sûr inférieur à D1 et une partie terminale 34 de diamètre D2. La partie terminale 34 comporte de préférence un bord extrême 36 biseauté.

Si l'on considère l'ensemble du canal 14, on trouve tout d'abord une portion 30 de diamètre D1 correspondant au diamètre de la chambre de pliage et donc au diamètre de l'implant plié, puis une portion 32 dont le diamètre interne va en diminuant jusqu'à la valeur D2, puis la partie terminale de diamètre D2. On comprend aisément qu'en poussant l'implant plié I à l'aide du piston 16 dans le canal 14, on fera passer son diamètre d'enroulement externe de D1 à D2.

Selon l'invention, on dispose, dans une position initiale à l'intérieur du canal 14, une pièce de poussée 40 interposée entre l'extrémité 16a du piston 16 et l'implant plié I initialement disposé dans la chambre d'enroulement. La poussée exercée par le piston 16 est ainsi transmise à l'implant plié 1 par l'intermédiaire de la pièce de poussée 40.

En se référant à la figure 3, on va décrire plus en détail la pièce de poussée 40. La pièce de poussée 40 présente une face latérale 42 de révolution autour de l'axe longitudinal X et X', une face antérieure 46 destinée à venir au contact de l'implant plié I et une face postérieure 44 destinée à venir au contact de l'extrémité 16a du piston 16. Les faces d'extrémité 44 et 46 sont sensiblement orthogonales à l'axe longitudinal X et X'. De plus, le diamètre de la face antérieure 46 référencé D5 est sensiblement égal au diamètre D1 de la partie courante du canal 14. En revanche la face postérieure 44 a un diamètre D4 supérieur au diamètre D1. En outre, la longueur L de la pièce de poussée 40 est sensiblement égale à la longueur de la portion de raccordement 32 du canal.

La pièce de poussée 40 est réalisée, de préférence entièrement, en un matériau élastiquement déformable et hydrophobe. Un matériau particulièrement adapté est le silicone.

On comprend que lorsque la pièce de poussée 40 est disposée à l'intérieur du canal 14, son extrémité postérieure de diamètre D4 est comprimée. Cette compression fait que lors de ses déplacements dans le canal 14, la pièce de poussée racle la face interne du canal et qu'elle crée un coefficient de frottement entre elle-même et la paroi du canal.

En outre, comme la pièce de poussée est hydrophobe, ses dimensions ne sont pas modifiées par le liquide présent dans l'injecteur.

De préférence, le diamètre D4 de la partie postérieure de la pièce de poussée 40 est compris entre 1,15 fois et 1,25 fois le diamètre D5 de sa face antérieure.

En conséquence, le diamètre externe D3 de la pièce de poussée 40 est au moins égal à D5, c'est-à-dire à D1.

En se référant maintenant aux figures 2A à 2D, on va décrire l'utilisation de l'injecteur d'implants par le chirurgien.

Sur la figure 2A, on a représenté de façon symbolique l'implant déjà plié I dans la chambre de pliage. Dans cette position initiale, la pièce de poussée 40 est disposée à l'entrée de la chambre de pliage 18 dans le canal 14. Du fait de sa forme générale tronconique, la pièce de poussée est maintenue en place dans le canal par la compression de sa partie postérieure. Lorsque le chirurgien exerce une poussée sur le piston 16, l'extrémité 16a de celui-ci vient au contact de la face postérieure 44 de la pièce de poussée 40 et provoque son déplacement dans la chambre de pliage puis dans la première partie 30 du canal. Lorsque la pièce de poussée 40 arrive dans la zone de raccordement 32 du canal, cette pièce peut aisément passer malgré le rétrécissement en raison de sa déformation élastique radiale. C'est ce que l'on a représenté sur la figure 2B. Le chirurgien continue à exercer une poussée sur le piston 16 de telle manière que la pièce de poussée 40 pénètre jusqu'à l'extrémité du canal, ce qui entraîne l'éjection contrôlée de l'implant, initialement plié, dans l'oeil du patient. Par ailleurs, du fait de la compression relativement importante de la pièce de poussée 40 dans la portion d'extrémité 32 et 34 du canal, cette pièce de poussée 40 reste coincée dans le canal lorsque le chirurgien procède au retrait du piston 16.

On comprend qu'ainsi grâce au coincement de la pièce de poussée 40 dans l'extrémité du canal de l'injecteur d'implants, cet injecteur ne peut en aucun cas être réutilisé pour la mise en place d'un autre implant souple.

Dans un mode préféré de réalisation, le diamètre D1 de la partie courante du canal, est égal sensiblement à 2 mm, le diamètre D2 de l'extrémité du canal est égal à 1,5 mm, le diamètre D5 de l'extrémité avant de la pièce de poussée 40 est égal à 2 mm alors que son extrémité postérieure 44 a un diamètre D4 égal à 2,2 mm en l'absence de contraintes. En outre, la longueur L de la pièce de poussée 40 est égale à 4 mm.

Il faut ajouter également que, du fait de la compression de la partie postérieure de la pièce de poussée dans le canal, le coefficient de frottement qui en résulte, permet au chirurgien d'exercer une poussée parfaitement contrôlée sur le piston 16 et donc de provoquer un déplacement de l'implant plié dans le canal régulier et sans à-coup, ce qui permet d'éviter une éjection intempestive de l'implant à l'intérieur de l'oeil du patient.

Il faut également ajouter que, du fait que l'extrémité antérieure 16a du piston 16 a un diamètre sensiblement égal à celui du canal, on évite des risques de coincement de la pièce de poussée 40 déformée entre le piston et la paroi interne du canal. De la même manière, du fait que la face antérieure 46 de la pièce de poussée qui est au contact de l'implant plié a un diamètre sensiblement égal à celui du canal, on évite que les anses haptiques de l'implant souple ne risquent de se coincer entre la paroi latérale de la pièce de poussée 40 et la paroi interne du canal 14.

## Revendications

1. Injecteur 10 d'implant intraoculaire souple comprenant :
- un canal (14) comportant une partie courante (30) de diamètre « D1 », une partie d'extrémité ouverte (34) de diamètre « D2 » (avec « D2 < D1 ») et une partie de raccordement (32) de forme sensiblement tronconique pour raccorder ladite partie courante à ladite partie d'extrémité ;
- un piston (16) rigide déplaçable dans le canal pour pousser l'implant plié dans le canal,
- une pièce de poussée (40) sensiblement de révolution, ladite pièce de poussée ayant, en l'absence de contraintes, un diamètre externe D3 au moins égal à « D1 »,
ledit injecteur est **caractérisé en ce que**
- le pièce de poussée sensiblement de révolution réalisée en un matériau hydrophobe et élastiquement déformable,
- ladite pièce étant disposée dans ledit canal entre l'implant (I) plié et l'extrémité dudit piston sans liaison mécanique avec ledit piston, par quoi, lorsque l'implant est poussé dans le canal à l'aide du piston, ladite pièce de poussée peut, par déformation élastique, pénétrer en partie dans la partie d'extrémité du canal en expulsant l'implant hors du canal, ladite pièce de poussée restant coincée dans ladite partie d'extrémité du canal lors du retrait du piston, rendant l'injecteur non réutilisable.

2. Injecteur selon la revendication 1, **caractérisé en ce que** l'extrémité de la pièce de poussée destinée à coopérer avec l'extrémité du piston a un diamètre « D4 > D1 » et **en ce que** l'autre extrémité de la pièce de poussée destinée à coopérer avec l'implant plié a un diamètre « D5 » sensiblement égal à « D1 », par quoi la déformation élastique radiale de la première extrémité de la pièce de poussée crée entre elle-même et la paroi interne du canal un coefficient de frottement.

3. Injecteur, selon la revendication 2, **caractérisé en ce que** le diamètre « D4 » de la première extrémité de la pièce de poussée est compris entre 1,15 fois et 1,25 fois le diamètre « D5 » de la deuxième extrémité.

4. Injecteur selon l'une quelconque des revendications 1 à 3, **caractérisé en ce que** ladite pièce de poussée est entièrement réalisée en silicone.

5. Injecteur selon l'une quelconque des revendications 1 à 4, **caractérisé en ce que** le diamètre D3 dudit piston est sensiblement égal à « D1 ».

6. Injecteur selon l'une quelconque des revendications 1 à 5, **caractérisé en ce que** la longueur axiale de ladite pièce de poussée est sensiblement égale à la longueur de la partie de raccordement du canal.

7. Injecteur selon l'une quelconque des revendications 1 à 6, **caractérisé en ce que** la face de la première extrémité de la pièce de poussée est sensiblement plane et orthogonale à l'axe longitudinale de la pièce de poussée.

8. Injecteur selon l'une quelconque des revendications 1 à 7, **caractérisé en ce que** ladite pièce de poussée est entièrement réalisée avec ledit matériau hydrophobe élastiquement déformable.

## Claims

1. An injector (10) for injecting a flexible intraocular implant, the injector comprising:
- a channel (14) having a main portion (30) of diameter D1, an open end portion (34) of diameter D2 (with D2 D1), and a joining portion (32) of substantially frustoconical shape for connecting said main portion to said end portion;
- a rigid piston (16) which is movable in the channel for pushing the folded implant through the channel;
- a pushing part (40) that is substantially rotationally symmetrical, said pushing part having an outer diameter D3 which is at least equal to D1, when no stress is applied;
said injector being **characterized in that**:
- said substantially rotationally symmetrical pushing part is made of a material that is hydrophobic and elastically deformable;
- said part is placed in said channel between the folded implant (I) and the end of said piston without being mechanically connected to said piston, whereby, when the implant is pushed along the channel by the piston, said pushing part may, due to its elastic deformation, partially enter into the end portion of the channel, thereby expelling the implant from the channel, said pushing part remaining jammed in said end portion of the channel, when the piston is withdrawn, thereby making the injector non-reusable.

2. The injector according to claim 1, **characterized in that** the end of the pushing part which is to co-operate with the end of the piston has a diameter D4 > D1 and **in that** the other end of the pushing part which is to co-operate with the folded implant has a diameter D5 which is substantially equal to D1, the radial elastic deformation of the first end of the pushing part thus creating a coefficient of friction between the pushing part and the inside wall of the channel.

3. The injector according to claim 2, **characterized in that** the diameter D4 of the first end of the pushing part is 1.15 times to 1.25 times the diameter D5 of the second end.

4. The injector according to any one of the claims 1 to 3, **characterized in that** said pushing part is entirely made of silicone.

5. The injector according to any one of the claims 1 to 4, **characterized in that** the diameter D3 of said piston is substantially equal to D1,

6. The injector according to any one of the claims I to 5, **characterized in that** the axial length of said pushing part is substantially equal to the length of the joining portion of the channel.

7. The injector according to any one of the claims I to 6, **characterized in that** the face of the first end of the pushing part is substantially plane and perpendicular to the longitudinal axis of the pushing part.

8. The injector according to any one the of claims 1 to 7, **characterized in that** said pushing part is entirely made of said elastically deformable hydrophobic material.

## Patentansprüche

1. Injektionsvorrichtung (10) für ein flexibles intraokulares Implantat, die Folgendes umfasst:
- einen Kanal (14), der einen Verlaufsabschnitt (30) mit einem Durchmesser "D1", einen offenen Endabschnitt (34) mit einem Durchmesser "D2" (wobei gilt: D2 < D1) und einen im Wesentlichen kegelstumpfförmigen Verbindungsabschnitt (32) zur Verbindung des Verlaufsabsclinitts und des Endabschnitts umfasst;
- einen starren Kolben (16), der in dem Kanal verschiebbar ist, um das gefaltete Implantat in den Kanal zu schieben,
- ein Schubstück (40), das im Wesentlichen rotationssymmetrisch ist, wobei das Schubstück, ohne Einwirkung von Belastungen, einen Außendurchmesser D3 aufweist, der zumindest "D1" entspricht,
wobei die Injektionsvorrichtung, **dadurch gekennzeichnet ist, dass**
- das im Wesentlichen rotationssymmetrische Schubstück aus einem hydrophoben und elastisch verformbaren Material besteht,
- das Schubstück in dem Kanal zwischen dem gefalteten Implantat (I) und dem Ende des Kolbens ohne mechanische Verbindung mit dem Kolben angeordnet ist, wodurch, wenn das Implantat mit Hilfe des Kolbens in den Kanal geschoben wird, das Schubstück durch elastische Verformung teilweise in den Endabschnitt des Kanals eindringen kann, wodurch das Implantat aus dem Kanal gedrückt wird, wobei das Schubstück in dem Endabschnitt des Kanals eingeklemmt bleibt, wenn der Kolben zurückgezogen wird, wodurch die Injektionsvorrichtung nicht wiederverwendbar ist.

2. Injektionsvorrichtung nach Anspruch 1, **dadurch gekennzeichnet, dass** das Ende des Schubstücks, das mit dem Ende des Kolbens in Kontakt gebracht werden soll, einen Durchmesser "D4 > D1" aufweist und dass das andere Ende des Schubstücks, das mit dem gefalteten Implantat zusammenwirken soll, einen Durchmesser "D5" aufweist, der im Wesentlichen mit "D1" übereinstimmt, wodurch die elastische radiale Verformung des ersten Endes des Schubstücks zwischen dem Schubstück und der Innenwand des Kanals einen Reibungskoeffzienten erzeugt.

3. Injektionsvorrichtung nach Anspruch 2, **dadurch gekennzeichnet, dass** der Durchmesser "D4" des ersten Endes des Schubstücks zwischen 1,15- und 1,25-mal so groß ist wie der Durchmesser "D5" des zweiten Endes.

4. Injektionsvorrichtung nach einem der Ansprüche 1 bis 3, **dadurch gekennzeichnet, dass** das Schubstück vollständig aus Silikon besteht.

5. Injektionsvorrichtung nach einem der Ansprüche 1 bis 4, **dadurch gekennzeichnet, dass** der Durchmesser "D3" des Kolbens im Wesentlichen "D1" entspricht.

6. Injektionsvorrichtung nach einem der Ansprüche 1 bis 5, **dadurch gekennzeichnet, dass** die Achsenlänge des Schubstücks im Wesentlichen der Länge des Verbindungsabschnitts des Kanales entspricht.

7. Injektionsvorrichtung nach einem der Ansprüche 1 bis 6, **dadurch gekennzeichnet, dass** die Fläche des ersten Endes des Schubstücks im Wesentlichen eben ist und im rechten Winkel auf die Längsachse des Schubstücks steht.

8. Injektionsvorrichtung nach einem der Ansprüche 1 bis 7, **dadurch gekennzeichnet, dass** das Schubstück vollständig aus dem hydrophoben, elastisch verformbaren Material besteht.
